Europäisches Patentamt

European Patent Office

Office européen des brevets

(19)

(11) Veröffentlichungsnummer: **0 264 804 B1**

(12) **EUROPÄISCHE PATENTSCHRIFT**

(45) Veröffentlichungstag der Patentschrift: **04.08.93**

(21) Anmeldenummer: **87114994.4**

(22) Anmeldetag: **14.10.87**

(51) Int. Cl.5: **C12N 11/00**, C12N 11/02, C07K 17/00, C07K 17/02, G01N 33/53, G01N 33/549, C12Q 1/00

(54) **Bioaffine poröse Festphasen, ein Verfahren zur Herstellung von bioaffinen porösen Festphasen und ihre Verwendung.**

(30) Priorität: **23.10.86 DE 3636060**

(43) Veröffentlichungstag der Anmeldung:
**27.04.88 Patentblatt 88/17**

(45) Bekanntmachung des Hinweises auf die Patenterteilung:
**04.08.93 Patentblatt 93/31**

(84) Benannte Vertragsstaaten:
**AT BE CH DE ES FR GB IT LI LU NL SE**

(56) Entgegenhaltungen:
EP-A- 0 063 810    EP-A- 0 097 952
EP-A- 0 200 381    DE-A- 3 329 728
US-A- 4 258 001    US-A- 4 366 241

**THE JOURNAL OF IMMUNOLOGY, Band 115, Nr. 6, Dezember 1975, Baltimore, USA, S.W. KESSLER "Rapid Isolation of Antigens from Cells with a Staphy-lococcal Protein A-Antibody Adsorbent: Parameters of the Interaction of Antibody-Antigen Complexes with Protein A", S. 1617-1624**

(73) Patentinhaber: **BEHRINGWERKE Aktiengesellschaft**
**Postfach 1140**
**W-3550 Marburg 1(DE)**

(72) Erfinder: **Friesen, Heinz-Jürgen, Dr.**
**Im Dorfe 4**
**W-3550 Marburg(DE)**

(74) Vertreter: **Meyer-Dulheuer, Karl-Hermann, Dr. et al**
**Hoechst AG, Werk Kalle-Albert, Zentrale Patentabteilung**
**W-6200 Wiesbaden (DE)**

**Beschreibung**

Die Erfindung betrifft eine bioaffine poröse Festphase und ein Verfahren zur Herstellung einer porösen Festphase, an die ein oder mehrere Bindungspartner eines bioaffinen Bindungssystems gebunden sind. Die Erfindung betrifft weiterhin die Verwendung von derart hergestellten porösen Festphasen zur Bindung der dem Bindungspartner entsprechenden Gegenpartner.

Es sind poröse Festphasen, an die Bindungspartner von bioaffinen Bindungssystemen gebunden sind, in einer Vielzahl bekannt, wobei die Bindungspartner Enzyme, Lectine, Antigene, Antikörper oder Partner anderer bioaffiner Systeme sind.

Beispielsweise sind poröse Festphasen mit bioaffinen Bindungspartnern in EPA 0063810 und in USP 4,366,241 beschrieben, bei denen die Bindungspartner adsorptiv oder kovalent mit dem Material der porösen Festphasen verbunden sind.

Weiterhin gibt es poröse Festphasen, die bioaffine Bindungspartner enthalten, bei denen Latexpartikel, an die Bindungspartner gekuppelt sind, in Filmschichten inkorporiert sind (EPA 97952 und DEOS 3329728).

Es wurde überraschenderweise gefunden, daß das Einbringen von Partikeln, an die ein oder mehrere verschiedene bioaffine Bindungspartner gekuppelt sind, in vorgeformte Materialien mit isotrop poröser, das heißt geschäumter oder anisotrop poröser, das heißt fasriger Struktur möglich ist in einer Weise, daß die Partikel in diesen Materialien fixiert werden.

Gegenstand der Erfindung ist eine poröse Festphase enthaltend einen bioaffinen Bindungspartner erhältlich dadurch, daß man in ein poröses aus Fasern bestehendes Material eine Dispersion oder Suspension von Partikeln, an die ein oder mehrere bioaffine Bindungspartner gebunden sind, einbringt und anschließend das Dispergierungs- oder Suspendierungsmittel entfernt.

Gegenstand der Erfindung ist auch ein Verfahren zur Herstellung von porösen Festphasen, enthaltend einen bioaffinen Bindungspartner, dadurch gekennzeichnet, daß man in ein poröses Material eine Dispersion oder Suspension von Partikeln, an die ein oder mehrere bioaffine Bindungspartner gebunden sind, einbringt und anschließend das Dispergierungs- oder Suspendierungsmittel entfernt.

Gegenstand der Erfindung ist weiterhin die Verwendung einer solchen porösen Festphase, enthaltend einen bioaffinen Bindungspartner, in Vorrichtungen zur Abtrennung und zum Nachweis des entsprechenden bioaffinen Gegenpartners sowie zum Nachweis des Bindungspartners in gelöster Form.

Das poröse Material kann in verschiedenen Formen ausgebildet sein. Beispielhaft sind Kugeln, Zylinder und flächenförmige Gebilde, wie Vliese und Membranen und Papiere.

Die porösen Strukturen können erzeugt werden durch Verschäumen oder durch Ausfällen mittels eines Fällbades von Lösungen oder Dispersionen synthetischer oder halbsynthetischer sowie natürlicher Polymere oder durch Verdunsten des Lösungsmittels aus Lösungen von membranbildenden Polymeren als auch durch Verpressen von Fasern, hergestellt aus den im folgenden genannten Polymeren.

Für die Herstellung der porösen Festphasen für diagnostische Mittel werden Vliese, Papiere oder Schwämme aus Zellulose oder Zellulosederivaten, sowie Membranen aus Zellulosederivaten oder Polyamiden bevorzugt.

Die Partikel können Latexpartikel sein, die ganz aus einem harten Polymeren oder im Kern aus einem harten Polymeren bestehen. Beispiele für solche Polymere sind durch Suspensions- oder Emulsionspolymerisation herstellbare Polymere wie Polystyrole oder Polyacrylate.

Bevorzugt sind Latices, die einen Kern-Schale-Aufbau mit hydrophiler Schale haben.

An der Oberfläche der Latices sind die bioaffinen Bindungspartner adsorptiv oder bevorzugt kovalent gebunden, wobei die Bindungspartner Rezeptoren, Lectine, Protein A von Staphylococcus aureus, Protein G von Streptococcus, Antigene, Haptene, Antikörper, Enzyme oder deren Inhibitoren sein können.

Die Partikel können auch aus stabilisierten Zellen oder Zellfragmenten bestehen.

Bevorzugt tragen solche Zellen oder Fragmente zu spezifischer Bindung befähigte Komponenten wie etwa Rezeptoren, die Antikörper über den $F_c$-Teil binden, davon beispielsweise Protein A von Staph. aureus oder Protein G von Streptococcus der Gruppe C oder G, Lectine, Antigene, Haptene, Antikörper, sowie über Protein A gebundene Antikörper, Enzyme oder deren Inhibitoren. Besonders bevorzugt sind ganze Zellen oder Membranfragmente von Staph. aureus, Stamm COWAN I, an die Antikörper gebunden sind wie von S.W.Kessler (J. Immunol., 1975, 115, 1617-1624) beschrieben.

Das erfindungsgemäße Verfahren kann ausgeführt werden, indem man Dispersionen und Suspensionen der Partikel auf die vorbeschriebenen porösen Materialien aufträgt oder durch Eintauchen einbringt und anschließend die dabei eingebrachte Flüssigkeit durch Verdunsten entfernt. Die Abstimmung von Poren- und Partikelgröße sowie Partikelmenge und Volumen der porösen Matrix erfolgt so, daß die für den jeweiligen Testaufbau erforderliche Flüssigkeitsströmung in der Matrix nicht behindert wird.

Bei Membranen werden Porengrößen von 0,1-12 μ und Partikelgrößen von 0,01-1 μ bevorzugt. Die aufgebrachten Partikeldispersionen oder Suspensionen haben eine Konzentration von 1-100 g/l. Das Aufgeben der Suspension auf den Träger kann durch Auftropfen, Aufpipettieren oder mit Hilfe von Pumpen und Kanülen punkt- oder strichförmig erfolgen. Das Trocknen kann durch Liegenlassen an der Luft, Überblasen von Luft bei Raumtemperatur oder erhöhter Temperatur in offenen oder geschlossenen Systemen bei Normaldruck oder im Vakuum erfolgen. Die Trocknungsbedingungen, insbesondere die erlaubte Thermobelastung werden durch die Stabilität der aktiven am Partikel gekoppelten Komponente bestimmt.

Die erfindungsgemäß hergestellten Festphasen können verwendet werden als biologisch aktive Festphasen wie etwa Enzym-, Antikörper-, Antigen- oder Hapten-Festphasen. Diese werden bevorzugt in diagnostischen Testelementen mit nebeneinander-, übereinander- oder gemischt neben- und übereinanderliegenden Zonen eingesetzt.

In den folgenden Beispielen wird die Herstellung von Latex- und zellulären Festphasen sowie deren Verwendung in diagnostischen Testelementen mit nebeneinanderliegenden Funktionszonen gezeigt. Die Festphasen sind dabei sowohl Enzym- als Antikörper-Festphasen.

Die Beispiele sind keineswegs als limitierend zu betrachten sondern dienen lediglich der weiteren Erläuterung des Erfindungsgegenstandes.

Beispiel 1

Glucoseoxidase enthaltende poröse Festphase

3 ml einer Dispersion, enthaltend 50 g/l Latex mit Acetalgruppen, hergestellt wie in Beispiel 2b von EPA 0 080 614 beschrieben, wurden mit wässrigen Lösungen von 300 μl 1 normaler HCl und von 300 μl 200 g/l (R)Tween 20 eine Stunde bei 20° C inkubiert. Es wurde dann durch Zugabe von 250 μl 1 normaler NaOH und gesättigter Lösung von $Na_2HPO_4$ ein pH von 6,5 eingestellt. Anschliessend wurden 1,5 ml einer Lösung von 1 g/l Glucoseoxidase mit einer Aktivität von 250 U/mg (Boehringer Mannheim, Reinheitsgrad 1, Best.Nr. 105 139) in phosphat-gepufferter physiologischer Kochsalzlösung, pH 7,2 (PBS) und 1,5 ml einer Lösung von 5 g/l Natriumcyanoborhydrid in PBS zugegeben und 15 h bei 4° C belassen. Dann wurden 1,2 ml 0,5 mol/l Äthanolamin, eingestellt auf pH 8,5 mit HCl und 0,3 ml einer Lösung von 25 g/l Natriumborhydrid zugegeben und das Gemisch eine weitere Stunde bei 4° C belassen. Nach Zentrifugation wurde das Sediment mit 2 g/l (R)Tween 20 in PBS resuspendiert, wobei ein Volumen von 15 ml der Dispersion von Latex-Glucoseoxidase Konjugat erhalten wurde.

Streifen von Filterpapier der Firma Macherey und Nagel, Düren, Bundesrepublik Deutschland, Prod.Nr. 215, wurden mit 40 μl/cm² der Dispersion von Latex-Glucoseoxidase Konjugat getränkt und anschließend bei 20-50° C getrocknet. In gleicher Weise wurden Membranen aus Zellulosemischester der Firma Millipore, USA, Prod.Nr. SCWP mit 10 μl/cm² der Dispersion getränkt und getrocknet.

Beispiel 2

Antikörper gegen humanes Myoglobin enthaltende poröse Festphase

2.1 Ein Konjugat von Latex und Kaninchen IgG mit Antikörpern gegen humanes Myoglobin wurde hergestellt, indem gemäß Beispiel 1 verfahren wurde mit der Veränderung, daß anstelle der Lösung von Glusoce-oxidase 1,5 ml einer Lösung von 0,2 mg/ml Kaninchen IgG mit Antikörpern gegen humanes Myoglobin verwandt wurde. Poröse Festphasen wurden hergestellt, wie in Beispiel 1 beschrieben.

2.2 Ein Konjugat von Zellen von Staphylococcus aureus (Stamm COWAN I) und Antikörper gegen humanes Myoglobin wurde wie folgt hergestellt:

Zu 50 ml einer 100 g/l Suspension von Staphylococcus aureus-Zellen in einer 9 g/l Natriumchlorid enthaltenden 0,05 mol/l wässrigen Natriumphosphatpufferlösung vom pH 7,4 wurden 16 ml einer Lösung von 2 g/l IgG vom Kaninchen, das Antikörper gegen humanes Myoglobin enthält, gegeben. Die Suspension wurde eine Stunde gerührt. Anschließend wurde die mit Antikörpern beladenen Zellen durch Zentrifugieren und Dekantieren vom Überstand abgetrennt. Die Zellen wurden in 45 ml des oben genannten Puffers resuspendiert und zur kovalenten Bindung des Antikörpers an den Zellen unter ständigem Rühren mit 50 ml einer Lösung von 2 g/l Glutardialdehyd in oben genanntem Puffer und nach einstündigem Rühren mit 50 ml einer Lösung von 50 g/l Natriumsulfit in dem oben genannten Puffer versetzt.

Nach einer weiteren Stunde Rühren wurden die Zellen durch Zentrifugieren und Dekantieren vom Überstand abgetrennt. Die Zellen wurden gewaschen, indem sie in 200 ml 0,05 mol/l wässrigen Natriumphosphatpuffer vom pH 7,4 resuspendiert wurden und durch Zentrifugieren und Dekantieren vom Waschpuffer abgetrennt wurden.

Die Festphasen wurden hergestellt, indem die so behandelten Zellen in PBS zu 20 g/l resuspendiert und wie in Beispiel 1 auf Filterpapier aufgetragen und eingetrocknet wurden.

Beispiel 3

Testelement zur Bestimmung von Myoglobin, enthaltend eine poröse Festphase gemäß Beispiel 2,1

3.1 Myoglobin-Peroxidase Konjugat

Es wurde elektrophoretisch einheitliches humanes Myoglobin und Peroxidase der Firma Boehringer Mannheim, Best.Nr. 413 470,verwandt. N-gamma-Maleinimidobutyryloxisuccinimid (GMBS) wurde von der Fa. Behring Diagnostics bezogen und wie von Tanimori et al., 1983, in J. Imm.Meth. 62, 123-131, beschrieben, mit humanem Myoglobin umgesetzt. 2-Iminothiolanhydrochlorid (Fa. Sigma, Kat.-Nr. I 6256) wurde wie von King et al., 1978, in Biochemistry 17, 1499-1506, beschrieben, mit Peroxidase umgesetzt. Aus dem Produkt der Umsetzung von GMBS mit humanem Myoglobin und der Iminothiolan-Peroxidase wurde ein Konjugat wie von Tanimori et al. beschrieben hergestellt. Das Rohkonjugat wurde durch Gelchromatographie an Ultrogel ACA 44 (Fa. LKB) gereinigt. Die Fraktion, in der etwa 1-2 Peroxidasemoleküle pro Molekül humanes Myoglobin gekoppelt waren, wurde für den Test verwendet. Das Konjugat wurde mit Enzygnost [R] IgE Inkubationsmedium der Behringwerke, Best.Nr. OSD auf 100 Pyrogalloleinheiten/ml verdünnt.

3.2 Herstellung der Bestandteile eines Testelements

3.2.1 Viskosevlies enthaltend Tetramethylbenzidin und Glucose

Viskosevlies mit einem Flächengewicht von 140-190 $g/m^2$ der Firma Kalle, Wiesbaden, Bundesrepublik Deutschland, wurde mit einer wässrigen Lösung, enthaltend 375 mg/l Tetramethylbenzidindihydrochlorid und 50 g/l D-Glucose getränkt in einer Weise, daß das Vlies keine weitere Menge der Lösung aufnehmen konnte. Das Vlies wurde bei 20-50° C getrocknet und in Plättchen der Größe 10x5 mm geschnitten.

3.2.2 Papier enthaltend Myoglobin-Peroxidase Konjugat und Glycoseoxidase

Ein auf 30x5 mm zurechtgeschnittenes Filterpapier Nr. 1 der Firma Macherey und Nagel wurde in 2 voneinander getrennten Bereichen einmal mit 5 μl einer Lösung Myoglobin-Peroxidase Konjugat gemäß Beispiel 3.1 und einmal mit 5 μl einer Lösung von 200 U/ml Glucoseoxidase getränkt, wobei die Auftragsstellen so gewählt wurden, daß die benetzten Bereiche des Papiers einen Abstand von 5-6 mm hatten. Das Filterpapier wurde bei 20-50° C getrocknet.

3.2.3 Papier enthaltend Antikörper gegen Myoglobin

Poröse Festphase gemäß Beispiel 2.1 wurde in Plättchen von 10x5 mm geschnitten.

3.2.4 Nicht imprägniertes Papier

Papier der Firma Schleicher und Schuell (Prod.Nr. 2668/8) wurde in rechteckige Scheibchen von 20x6 mm geschnitten.

4.3 Herstellung des Testelements

Auf eine Polyesterfolie wurde mit Hilfe eines doppelseitig klebenden Bandes jeweils eines der rechteckigen Plättchen über ihre Schmalseiten in saugfähigem Kontakt hintereinander aufgeklebt in der Reihenfolge:

a) Viskosevlies, gemäß Beispiel 3.2.1,

b) Papier, gemäß Beispiel 3.2.2 derart positioniert, daß der die Glucoseoxidase enthaltende Bereich an die poröse Festphase anschloß,

c) Poröse Festphase, gemäß Beispiel 3.2.3 und

d) Papier gemäß Beispiel 3.2.4

4.4 Funktionsprüfung des Testelements

Bei Auftragen von jeweils 100 μl einer Lösung von 10, 100, 1000 und 10 000 μg/ml humanem Myoglobin in Enzygnost[R] IgE Verdünnungspuffer auf das Viskosevlies eines jeweiligen Testelements wurden nach 12 bis 13 min die Farbintensitäten, die auf der porösen Festphase entstanden, mit dem für den Gehalt an Glucose im Blut geeichten Reflexionsphotometer [R]Sanoquell der Firma Quelle, gemessen und folgende Meßwerte erhalten:

| Myoglobin | Meßsignal |
| :---: | :---: |
| | geeicht für |
| µg/ml | mg Glucose pro dl Blut |
| 10 | 115 |
| 100 | 110 |
| 1000 | 70 |
| 10000 | 0 |

Ein in gleicher Weise funktionsfähiges Testelement konnte hergestellt werden, wenn an Latex gebundene Glucoseoxidase gemäß Beispiel 1 als poröse Festphase, wie in Beispiel 3.2.2 beschrieben, fixiert und auch wenn der Antikörper an Zellen von Staphylococcus aureus gebunden und in der porösen Festphase entsprechend Beispiel 2.2 fixiert worden war.

**Patentansprüche**
**Patentansprüche für folgende Vertragsstaaten : AT, BE, CH, DE, FR, GB, IT, LI, LU, NL, SE**

1. Poröse Festphase, die aus einem vorgeformten porösen Material und darin durch Tränken oder Auftragen eingebrachten Partikeln besteht, die mit einem oder mehreren bioaffinen Bindungspartnern gekoppelt sind, dadurch gekennzeichnet, daß als Partikel Latexpartikel, die ganz aus einem harten Polymeren oder im Kern aus einem harten Polymeren bestehen, oder stabilisierte Zellen oder Zellfragmente eingesetzt werden und daß das poröse Material ein Papier ist.

2. Poröse Festphase nach Anspruch 1, dadurch gekennzeichnet, daß die Partikel Zellen von Staphylococcus aureus des Stammes COWAN I sind und Immunglobulin an die Partikel gebunden wurde.

3. Poröse Festphase nach Anspruch 1, dadurch gekennzeichnet, daß die Partikel mit Protein A von Staphylococcus aureus beschichtet wurden.

4. Poröse Festphase nach Anspruch 1, dadurch gekennzeichnet, daß die Partikel mit Protein G von Streptococcus der Gruppe C oder G beschichtet und Immunglobulin an die Partikel gebunden wurde.

5. Verfahren zur Herstellung einer porösen Festphase nach mindestens einem der Ansprüche 1 bis 4, dadurch gekennzeichnet, daß man in das vorgeformte Material eine Dispersion oder Suspension von Partikeln, an die ein oder mehrere bioaffine Bindungspartner gebunden sind, einbringt und anschließend das Dispergierungs- oder Suspendierungsmittel entfernt.

6. Verwendung einer porösen Festphase nach mindestens einem der Ansprüche 1 bis 4 in Vorrichtungen zur Abtrennung und zum Nachweis des entsprechenden bioaffinen Gegenpartners.

7. Poröse Festphase nach mindestens einem der Ansprüche 1 - 4, dadurch gekennzeichnet, daß die Porengröße des vorgeformten porösen Materials 0,1 - 12 µm und die Partikelgröße 0,01 - 1 µm beträgt.

**Patentansprüche für folgenden Vertragsstaat : ES**

1. Poröse Festphase, die aus einem vorgeformten porösen Material und darin durch Tränken oder Auftragen eingebrachten Partikeln besteht, die mit einem oder mehreren bioaffinen Bindungspartnern gekoppelt sind, dadurch gekennzeichnet, daß als Partikel Latexpartikel, die ganz aus einem harten Polymeren oder im Kern aus einem harten Polymeren bestehen, oder stabilisierte Zellen oder

EP 0 264 804 B1

Zellfragmente eingesetzt werden.

2. Poröse Festphase nach Anspruch 1, dadurch gekennzeichnet, daß das poröse Material ein Papier ist.

3. Poröse Festphase nach Anspruch 1, dadurch gekennzeichnet, daß das poröse Material ein Vlies ist.

4. Poröse Festphase nach Anspruch 1, dadurch gekennzeichnet, daß die Partikel Zellen von Staphylococcus aureus des Stammes COWAN I sind und Immunglobulin an die Partikel gebunden wurde.

5. Poröse Festphase nach Anspruch 1, dadurch gekennzeichnet, daß die Partikel mit Protein A von Staphylococcus aureus beschichtet wurden.

6. Poröse Festphase nach Anspruch 1, dadurch gekennzeichnet, daß die Partikel mit Protein G von Streptococcus der Gruppe C oder G beschichtet und Immunglobulin an die Partikel gebunden wurde.

7. Verfahren zur Herstellung einer porösen Festphase nach mindestens einem der Ansprüche 1 bis 6, dadurch gekennzeichnet, daß man in das vorgeformte Material eine Dispersion oder Suspension von Partikeln, an die ein oder mehrere bioaffine Bindungspartner gebunden sind, einbringt und anschließend das Dispergierungs- oder Suspendierungsmittel entfernt.

8. Verwendung einer porösen Festphase nach mindestens einem der Ansprüche 1 bis 6 in Vorrichtungen zur Abtrennung und zum Nachweis des entsprechenden bioaffinen Gegenpartners.

9. Poröse Festphase nach mindestens einem der Ansprüche 1 - 6, dadurch gekennzeichnet, daß die Porengröße des vorgeformten porösen Materials 0,1 - 12 $\mu$m und die Partikelgröße 0,01 - 1 $\mu$m beträgt.

**Claims**
**Claims for the following Contracting States : AT, BE, CH, DE, FR, GB, IT, LI, LU, NL, SE**

1. A porous solid phase which is composed of a preshaped porous material and of particles which have been introduced therein by impregnation or application and which are coupled to one or more binding partners having bioaffinity, wherein latex particles which are composed of a rigid polymer entirely or of a rigid polymer in the core, or stabilized cells or cell fragments are employed as particles, and wherein the porous material is a paper.

2. A porous solid phase as claimed in claim 1, wherein the particles are cells of Staphylococcus aureus of the COWAN I strain, and immunoglobulin has been bound to the particles.

3. A porous solid phase as claimed in claim 1, wherein the particles have been coated with protein A from Staphylococcus aureus.

4. A porous solid phase as claimed in claim 1, wherein the particles have been coated with protein G from Streptococcus of group C or G, and immunoglobulin has been bound to the particles.

5. A process for preparing a porous solid phase as claimed in at least one of claims 1 to 4, which comprises introducing into the preshaped material a dispersion or suspension of particles to which one or more binding partners having bioaffinity are bound, and subsequently removing the dispersing or suspending agent.

6. The use of a porous solid phase as claimed in at least one of claims 1 to 4 in apparatuses for removal and detection of the corresponding counter-partner having bioaffinity.

7. A porous solid phase as claimed in at least one of claims 1 - 4, wherein the pore size of the preshaped porous material is 0.1 - 12 $\mu$m and the particle size is 0.01 - 1 $\mu$m.

6

**Claims for the following Contracting State : ES**

1.  A porous solid phase which is composed of a preshaped porous material and of particles which have been introduced therein by impregnation or application and which are coupled to one or more binding partners having bioaffinity, wherein latex particles which are composed of a rigid polymer entirely or of a rigid polymer in the core, or stabilized cells or cell fragments are employed as particles.

2.  A porous solid phase as claimed in claim 1, wherein the porous material is a paper.

3.  A porous solid phase as claimed in claim 1, wherein the porous material is a nonwoven fabric.

4.  A porous solid phase as claimed in claim 1, wherein the particles are cells of Staphylococcus aureus of the COWAN I strain, and immunoglobulin has been bound to the particles.

5.  A porous solid phase as claimed in claim 1, wherein the particles have been coated with protein A from Staphylococcus aureus.

6.  A porous solid phase as claimed in claim 1, wherein the particles have been coated with protein G from Streptococcus of group C or G, and immunoglobulin has been bound to the particles.

7.  A process for preparing a porous solid phase as claimed in at least one of claims 1 to 6, which comprises introducing into the preshaped material a dispersion or suspension of particles to which one or more binding partners having bioaffinity are bound, and subsequently removing the dispersing or suspending agent.

8.  The use of a porous solid phase as claimed in at least one of claims 1 to 6 in apparatuses for removal and detection of the corresponding counter-partner having bioaffinity.

9.  A porous solid phase as claimed in at least one of claims 1 - 6, wherein the pore size of the preshaped porous material is 0.1 - 12 $\mu$m and the particle size is 0.01 - 1 $\mu$m.

**Revendications**
**Revendications pour les Etats contractants suivants : AT, BE, CH, DE, FR, GB, IT, LI, LU, NL, SE**

1.  Phase solide poreuse, constituée d'un matériau poreux préformé et de particules qu'on y a introduites, par impregnation ou par application, et qui sont couplées à un ou plusieurs partenaires de liaison bio-affins, caractérisée en ce que, comme particules, on utilise des particules de latex constituées, intégralement ou pour leur partie centrale, d'un polymère rigide, ou des cellules ou fragments cellulaires stabilisés, et le matériau poreux est un papier.

2.  Phase solide poreuse selon la revendication 1, caractérisée en ce que les particules sont des cellules de Staphylococcus aureus de la souche COWAN I et on a lié une immunoglobuline aux particules.

3.  Phase solide poreuse selon la revendication 1, caractérisée en ce que l'on a recouvert les particules de protéine A de Staphylococcus aureus.

4.  Phase solide poreuse selon la revendication 1, caractérisée en ce que l'on a recouvert les particules de protéine G de Streptococcus du groupe C ou G, et on a lié une immunoglobuline aux particules.

5.  Procédé pour préparer une phase solide poreuse selon au moins une des revendications 1 à 4, caractérisé en ce que l'on introduit une dispersion ou une suspension de particules, auxquelles sont liés un ou plusieurs partenaires bio-affins, dans le matériau préformé, et on élimine ensuite l'agent dispersant ou l'agent de mise en suspension.

6.  Utilisation d'une phase solide poreuse selon au moins une des revendications 1 à 4 dans des dispositifs destinés à séparer et à identifier le partenaire bio-affin opposé correspondant.

**7.** Phase solide poreuse selon au moins une des revendications 1 à 4, caractérisée en ce que les pores du matériau poreux préformé ont une dimension valant de 0,1 à 12 $\mu$m et les particules ont une dimension valant de 0,01 à 1 $\mu$m.

**Revendications pour l'Etat contractant suivant : ES**

**1.** Phase solide poreuse, constituée d'un matériau poreux préformé et de particules qu'on y a introduites, par impregnation ou par application, et qui sont couplées à un ou plusieurs partenaires de liaison bio-affins, caractérisée en ce que, comme particules, on utilise des particules de latex constituées, intégralement ou pour leur partie centrale, d'un polymère rigide, ou des cellules ou fragments cellulaires stabilisés.

**2.** Phase solide poreuse selon la revendication 1, caractérisée en ce que le matériau poreux est un papier.

**3.** Phase solide poreuse selon la revendication 1, caractérisée en ce que le matériau poreux est un tissu non-tissé.

**4.** Phase solide poreuse selon la revendication 1, caractérisée en ce que les particules sont des cellules de Staphylococcus aureus de la souche COWAN I et on a lié une immunoglobuline aux particules.

**5.** Phase solide poreuse selon la revendication 1, caractérisée en ce que l'on a recouvert les particules de protéine A de Staphylococcus aureus.

**6.** Phase solide poreuse selon la revendication 1, caractérisée en ce que l'on a recouvert les particules de protéine G de Streptococcus du groupe C ou G, et on a lié une immunoglobuline aux particules.

**7.** Procédé pour préparer une phase solide poreuse selon au moins une des revendications 1 à 6, caractérisé en ce que l'on introduit une dispersion ou une suspension de particules, auxquelles sont liés un ou plusieurs partenaires bio-affins, dans le matériau préformé, et on élimine ensuite l'agent dispersant ou l'agent de mise en suspension.

**8.** Utilisation d'une phase solide poreuse selon au moins une des revendications 1 à 6 dans des dispositifs destinés à séparer et à identifier le partenaire bio-affin opposé correspondant.

**9.** Phase solide poreuse selon au moins une des revendications 1 à 6, caractérisée en ce que les pores du matériau poreux préformé ont une dimension valant de 0,1 à 12 $\mu$m et les particules ont une dimension valant de 0,01 à 1 $\mu$m.